# EUROPEAN PATENT SPECIFICATION

(11) **EP 2 960 325 B1**
(45) Date of publication and mention of the grant of the patent: **27.09.2017**
(21) Application number: 15177618.4
(22) Date of filing: 26.01.2001
(51) Int. Cl.: A23D 9/00, C12N 1/00, C12P 7/64

(54) **ENHANCED PRODUCTION OF LIPIDS CONTAINING POLYENOIC FATTY ACIDS BY HIGH DENSITY CULTURES OF EUKARYOTIC MICROBES IN FERMENTORS**
VERSTÄRKTE PRODUKTION VON LIPIDEN ENTHALTEND MEHRFACH UNGESAETTIGTE FETTSAEUREN DURCH HOCHDICHTE KULTUREN VON EUKARYOTISCHEN MIKROORGANISMEN IN FERMENTOREN
PRODUCTION AMELIORÉE DE LIPIDES CONTENANT DES ACIDES GRAS POLYÈNES AU MOYEN DE CULTURES À GRANDE DENSITÉ DE MICROBES EUCARYOTES DANS DES FERMENTEURS

(30) Priority: 28.01.2000 US 178588 P
(43) Date of publication of application: 30.12.2015
(62) Divisional of application: 10012184.7
(73) Proprietor: DSM IP Assets B.V., 6411 TE Heerlen (NL)
(72) Inventor: BAILEY, Richard B., Columbia, MD Maryland 21045 (US); DIMASI, Don, Columbia, MD Maryland 21045 (US); HANSEN, Jon Milton, West Friendship, MD Maryland 21794 (US); MIRRASOUL, Peter J., San Diego, CA California 92107 (US); RUECKER, Craig M., Columbia, MD Maryland 21045 (US); VEEDER, George T. III, Case Grande, AZ 85122 (US); KANEKO, Tatsuo, San Diego, CA California 92126 (US); BARCLAY, William R., Boulder, CO Colorado 80303 (US)
(74) Representative: Hoyng Rokh Monegier LLP

(56) References cited:
- EP-A1- 0 823 475
- WO-A-94/08467
- WO-A2-98/37179
- US-A- 4 783 408
- MEESTERS P A E P ET AL: "High-cell-density cultivation of the lipid accumulating yeastusing glycerol as a carbon source", APPLIED MICROBIOLOGY AND BIOTECHNOLOGY, SPRINGER, BERLIN, DE, vol. 45, no. 5, 1 June 1996 (1996-06-01), pages 575-579, XP035171368, ISSN: 1432-0614, DOI: 10.1007/S002530050731
- LENA HANSSON ET AL: "Influence of cultivation conditions on lipid production by Cryptococcus albidus", APPLIED MICROBIOLOGY AND BIOTECHNOLOGY, SPRINGER, DE, vol. 24, no. 1, 1 April 1986 (1986-04-01), pages 12-18, XP035171225, ISSN: 0175-7598, DOI: 10.1007/BF00266278
- LENA HANSSON ET AL: "Lipid formation by Cryptococcus albidus in nitrogen-limited and in carbon-limited chemostat cultures", APPLIED MICROBIOLOGY AND BIOTECHNOLOGY, SPRINGER, BERLIN, DE, vol. 24, no. 3, 1 June 1986 (1986-06-01), pages 187-192, XP035170645, ISSN: 1432-0614, DOI: 10.1007/BF00261535

## Description

### FIELD OF THE INVENTION

The present invention is directed to a novel process for growing microorganisms of the order of Thraustochytriales that are capable of producing at least 20% of their biomass as lipids comprising some polyenoic fatty acids.

### BACKGROUND OF THE INVENTION

Production of polyenoic fatty acids (fatty acids containing 2 or more unsaturated carbon-carbon bonds) in eukaryotic microorganisms has been generally believed to require the presence of molecular oxygen (*i.e*., aerobic conditions). This is because it is believed that the cis double bond formed in the fatty acids of all non-parasitic eukaryotic microorganisms involves a direct oxygen-dependent desaturation reaction (oxidative microbial desaturase systems). Other eukaryotic microbial lipids that are known to require molecular oxygen include fungal and plant sterols, oxycarotenoids (*i.e*., xanthophylls), ubiquinones, and compounds made from any of these lipids (*i.e*., secondary metabolites).

Certain eukaryotic microbes (such as algae; fungi, including yeast; and protists) have been demonstrated to be good producers of polyenoic fatty acids in fermentors. However, very high density cultivation (greater than about 100 g/L microbial biomass, especially at commercial scale) can lead to decreased polyenoic fatty acid contents and hence decreased polyenoic fatty acid productivity. This may be due in part to several factors including the difficulty of maintaining high dissolved oxygen levels due to the high oxygen demand developed by the high concentration of microbes in the fermentation broth. Methods to maintain higher dissolved oxygen level include increasing the aeration rate and/or using pure oxygen instead of air for aeration and/or increasing the agitation rate in the fermentor. These solutions generally increase the cost of lipid production and capital cost of fermentation equipment, and can cause additional problems. For example, increased aeration can easily lead to severe foaming problems in the fermentor at high cell densities and increased mixing can lead to microbial cell breakage due to increased shear forces in the fermentation broth (this causes the lipids to be released in the fermentation broth where they can become oxidized and/or degraded by enzymes). Microbial cell breakage is an increased problem in cells that have undergone nitrogen limitation or depletion to induce lipid formation, resulting in weaker cell walls.

As a result, when lipid-producing eukaryotic microbes are grown at very high cell concentrations, their lipids generally contain only very small amounts of polyenoic fatty acids. For example, the yeast *Lipomyces starkeyi* has been grown to a density of 153 g/L with resulting lipid concentration of 83 g/L in 140 hours using alcohol as a carbon source. Yet the polyenoic fatty acid content of the yeast at concentration greater than 100 g/L averaged only 4.2% of total fatty acids (dropping from a high of 11.5% of total fatty acid at a cell density of 20-30 g/L). Yamauchi et al., J. Ferment. Technol., 1983, 61, 275-280. This results in a polyenoic fatty acid concentration of only about 3.5 g/L and an average polyenoic fatty acid productivity of only about 0.025 g/L/hr. Additionally, the only polyenoic fatty acid reported in the yeast lipids was C18:2.

Another yeast, *Rhodotorula glutinus,* has been demonstrated to have an average lipid productivity of about 0.49 g/L/hr, but also a low overall polyenoic fatty acid content in its lipids (15.8% of total fatty acids, 14.7% C18:2 and 1.2% C18:3) resulting in a polyenoic fatty acid productivity in fed-batch culture of only about 0.047 g/L/hr and 0.077 g/L/hr in continuous culture.

One of the present inventors has previously demonstrated that certain marine microalgae in the order Thraustochytriales can be excellent producers of polyenoic fatty acids in fermentors, especially when grown at low salinity levels and especially at very low chloride levels. Others have described Thraustochytrids that exhibit an average polyenoic fatty acid (DHA, C22:6n-3; and DPA, C22:5n-6) productivity of about 0.158 g/L/hr, when grown to cell density of 59 g/L in 120 hours. However, this productivity was only achieved at a salinity of about 50% seawater, a concentration that would cause serious corrosion in conventional stainless steel fermentors.

Meesters et al, Applied microbiology and biotechnology, Vol. 45, No. 5 (1996) 575-579 describes high-cell-density cultivation of the lipid accumulating yeast *Cryptococcus curvatus* using glycerol as a carbon source.

US 4,783,408 describes a method for the preparation of γ-linolenic acid, comprising culturing a fungus of *Mortierella* genus including *isabellina, vinacea, ramanniana, ramanniana* var. angulispora and *nana.*

Lena Hansson et al., Appl. Microbiol Biotechnol (1986) 24 : 12-18 describes the influence of cultivation conditions on lipid production by the yeast *Cryptococcus albidus.*

Lena Hansson et al., Appl. Microbiol Biotechnol (1986) 24 : 187 - 192 describes lipid formation by the yeast *Cryptococcus albidus* in nitrogen-limited and in carbon-limited chemostat cultures.

EP 0 823 475 A1 describes the cultivation of the *Schizochytrium* SR21 strain having the ability to produce a fat containg the (n-3) series of docosahexaenoic acid and the (n-6) series of docosapentaenoic acid.

Costs of producing microbial lipids containing polyenoic fatty acids, and especially the highly unsaturated fatty acids, such as C18:4n-3, C20:4n-6, C20:5n3, C22:5n-3, C22:5n-6 and C22:6n-3, have remained high in part due to the limited densities to which the high polyenoic fatty acid containing eukaryotic microbes have been grown and the limited oxygen availability both at these high cell concentrations and the higher temperatures needed to achieve high productivity.

Therefore, there is a need for a process for growing microorganisms at high concentration which still facilitates increased production of lipids containing polyenoic fatty acids.

### SUMMARY OF THE INVENTION

The present invention provides a process for growing eukaryotic microorganisms of the order Thraustochytriales that are capable of producing at least 20% of their biomass as lipids and a method for producing the lipids. The lipids contain one or more polyenoic fatty acids. The process comprises adding to a fermentation medium comprising eukaryotic microorganisms of the order Thraustochytriales a carbon source, preferably a non-alcohol carbon source, and a limiting nutrient source. The carbon source and the limiting nutrient source are added at a rate sufficient to increase the biomass density of the fermentation medium to at least 100 g/L.

The fermentation condition may comprise a biomass density increasing stage and a lipid production stage, wherein the biomass density increasing stage comprises adding the carbon source and the limiting nutrient source, and the lipid production stage comprises adding the carbon source without adding the limiting nutrient source to create conditions which induce lipid production.

In an embodiment the amount of dissolved oxygen present in the fermentation medium during the lipid production stage is lower than the amount of dissolved oxygen present in the fermentation medium during the biomass density increasing stage.

In an embodiment of the present invention, microorganisms are grown in a fed-batch process.

In an embodiment the present invention provides maintaining an oxygen level of less than 3% of saturation in the fermentation medium during the second half of the fermentation process.

There is also described a process for producing eukaryotic microbial lipids comprising:
(a) growing eukaryotic microorganisms in a fermentation medium to increase the biomass density of said fermentation medium to at least about 100 g/L;
(b) providing fermentation conditions sufficient to allow said microorganisms to produce said lipids; and
(c) recovering said lipids,
wherein greater than about 15% of said lipids are polyunsaturated lipids.

Another embodiment provides a lipid recovery process that comprises:
(d) removing water from said fermentation medium to provide dry microorganisms; and
(e) isolating said lipids from said dry microorganisms.

Preferably, the water removal step comprises contacting the fermentation medium directly on a drum-dryer without prior centrifugation.

Another aspect provides a lipid recovery process that comprises:
(d) treating the fermentation broth to permeabilize, lyse or rupture the microbial cells; and
(e) recovering the lipids from the fermentation broth by gravity separation, and preferably centrifugation, with or without the aid of a water-soluble solvent to aid in breaking the lipid/water emulsion.

Preferably, the microbial cells are treated in step (c) in a fermentor or a similar vessel.

### BRIEF DESCRIPTION OF THE DRAWINGS

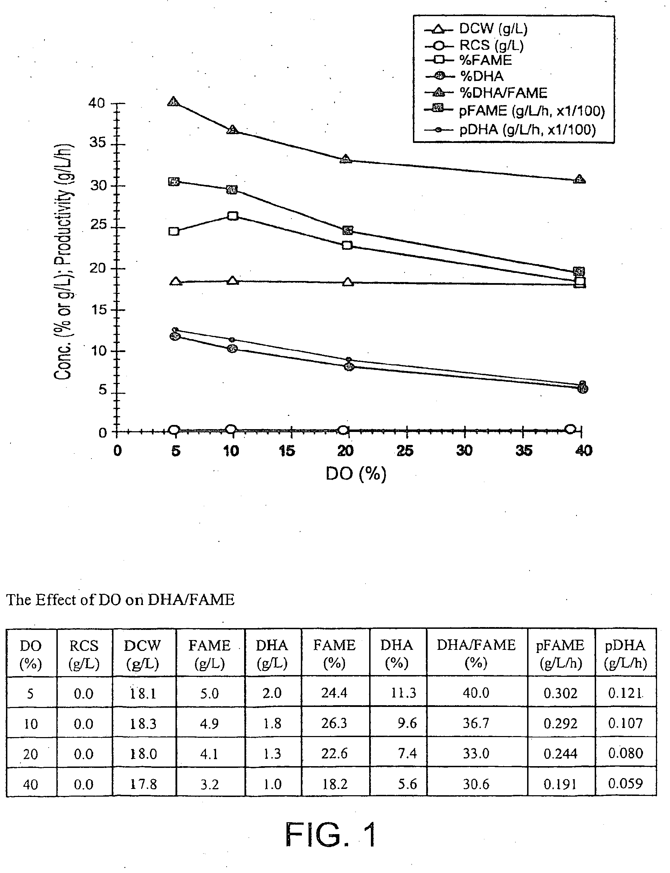

Figure 1 is a table and a plot of various lipid production parameters of a microorganism versus the amount of dissolved oxygen in a fermentation medium.

### DETAILED DESCRIPTION OF THE INVENTION

The process of the present invention can be used to produce highly unsaturated lipids (*i.e*., lipids containing 4 or more unsaturated carbon-carbon bonds) such as omega-3 and/or omega-6 polyunsaturated fatty acids, including docosahexaenoic acid (*i.e*., DHA); and other naturally occurring unsaturated, polyunsaturated and highly unsaturated compounds. As used herein, the term "lipid" includes phospholipids; free fatty acids; esters of fatty acids; triacylglycerols; sterols and sterol esters; carotenoids; xanthophylls (*e.g*., oxycarotenoids); hydrocarbons; isoprenoid-derived compounds and other lipids known to one of ordinary skill in the art.

Specifically, processes of the present invention are useful in growing microorganisms of the order Thraustochytriales that produce polyenoic fatty acid(s), and for producing microbial polyenoic fatty acid(s).

While processes of the present invention can be used to grow a wide variety of microorganisms of the order Thraustochytriales and to obtain polyunsaturated lipid containing compounds produced by the same, for the sake of brevity, convenience and illustration, this detailed description of the invention will discuss processes for growing microorganisms of the order Thraustochytriales which are capable of producing lipids comprising omega-3 and/or omega-6 polyunsaturated fatty acids, in particular microorganisms that are capable of producing DHA (or closely related compounds such as DPA, EPA or ARA).

More particularly, preferred embodiments of the present invention will be discussed with reference to a process for growing marine microorganisms of the order Thraustochytriales, more specifically Thraustochytriales of the genus *Thraustochytrium* and *Schizochytrium,* including Thraustochytriales which are disclosed in commonly assigned U.S. Patent Nos. 5,340,594 and 5,340,742, both issued to Barclay. It should be noted that many experts agree that *Ulkenia* is not a separate genus, but is in fact part of the genus *Schizochytrium.* As used herein, the genus *Schizochytrium* will include *Ulkenia.*

Preferred microorganisms are those that produce the compounds of interest via polyketide synthase systems. Such microorganisms include microorganisms having an endogenous polyketide synthase system and microorganisms into which a polyketide synthase system has been genetically engineered. Polyketides are structurally diverse natural products that have a wide range of biological activities, including antibiotic and pharmacological properties. Biosynthesis of the carbon chain backbone ofpolyketides is catalyzed by polyketide synthases. Like the structurally and mechanistically related fatty acid synthases, polyketide synthases catalyze the repeated decarboxylative condensations between acyl thioesters that extend the carbon chain two carbons at a time. However, unlike fatty acid synthases, polyketide synthases can generate great structural variability in the end product. Individual polyketide synthase systems can do this by using starting units other than acetate, by utilizing methyl- or ethyl-malonate as the extending unit and by varying the reductive cycle of ketoreduction, dehydration and enoyl reduction on the beta-keto group formed after each condensation. Of particular interest here is that the carbon-carbon double bonds that are introduced by the dehydration step can be retained in the end product. Further, although these double bonds are initially in the *trans* configuration, they can be converted to the *cis* configuration found in DHA (and other polyenoic fatty acids of interest) by enzymatic isomerization. Both the dehydrase and isomerization reactions can occur in the absence of molecular oxygen.

Preferably, in accordance with the present invention a heterotrophic process is provided for producing products and microorganisms. The process preferably comprises culturing the microorganisms in a growth medium wherein the microorganisms contain a polyketide synthase system. Preferably, the level of dissolved oxygen is maintained at less than about 8 percent, more preferably at less than about 4 percent, more preferably at less than about 3 percent, and more preferably at less than about 1 percent.

Assuming a relatively constant production rate of lipids by an algae, it is readily apparent that the higher biomass density will lead to a higher total amount of lipids being produced per volume. Current conventional fermentation processes for growing algae yield a biomass density of from about 50 to about 80 g/L or less. The present inventors have found that by using processes of the present invention, a significantly higher biomass density than currently known biomass density can be achieved. The process of the present invention produces biomass density of at least 100 g/L, more preferably at least about 130 g/L, still more preferably at least about 150 g/L, yet still more preferably at least about 170 g/L, and most preferably greater than 200 g/L. Thus, with such a high biomass density, even if the lipids production rate of algae is decreased slightly, the overall lipids production rate per volume is significantly higher than currently known processes.

Processes of the present invention for growing microorganisms of the order Thraustochytriales include adding a source of carbon and a source of a limiting nutrient to a fermentation medium comprising the microorganisms at a rate sufficient to increase the biomass density of the fermentation medium to those described above. As used herein, the term "limiting nutrient source" refers to a source of a nutrient (including the nutrient itself) essential for the growth of a microorganism in that, when the limiting nutrient is depleted from the growth medium, its absence substantially limits the microorganism from growing or replicating further. However, since the other nutrients are still in abundance, the organism can continue to make and accumulate intracellular and/or extracellular products. By choosing s specific limiting nutrient, one can control the type of products that are accumulated. Therefore, providing a limiting nutrient source at a certain rate allows one to control both the rate of growth of the microorganism and the production or accumulation of desired products (e.g., lipids). This fermentation process, where one or more substrates (*e.g*., a carbon source and a limiting nutrient source) are added in increments, is generally referred to as a fed-batch fermentation process. It has been found that when the substrate is added to a batch fermentation process the large amount of carbon source present (*e.g*., about 200 g/L or more per 60 g/L of biomass. density) had a detrimental effect on the microorganisms. Without being bound by any theory, it is believed that such a high amount of carbon source causes detrimental effects, including osmotic stress, for microorganisms and inhibits initial productivity of microorganisms. Processes of the present invention avoid this undesired detrimental effect while providing a sufficient amount of the substrate to achieve the above-described biomass density of the microorganisms.

Processes of the present invention for growing microorganisms include a biomass density increasing stage. In the biomass density increasing stage, the primary objective of the fermentation process is to increase the biomass density in the fermentation medium to obtain the biomass density described above. The rate of carbon source addition is typically maintained at a particular level or range that does not cause a significant detrimental effect on productivity of microorganisms, or the viability of the microorganisms resulting from insufficient capabilities of the fermentation equipment to remove heat from and transfer gases to and from the liquid broth An appropriate range of the amount of carbon source needed for a particular microorganism during a fermentation process is well known to one of ordinary skill in the art. Preferably, a carbon source of the present invention is a non-alcohol carbon source, *i.e*., a carbon source that does not contain alcohol. As used herein, an "alcohol" refers to a compound having 4 or less carbon atoms with one hydroxy group, *e.g.*, methanol, ethanol and isopropanolbut for the purpose of this invention does not include hydroxy organic acids such as lactic acid and similar compounds. More preferably, a carbon source of the present invention is a carbohydrate, including, but not limited to, fructose, glucose, sucrose, molasses, and starch. Other suitable simple and complex carbon sources and nitrogen sources are disclosed in the above-referenced patents. Typically, however, a carbohydrate, preferably com syrup, is used as the primary carbon source. Fatty acids, in the form of hydroxy fatty acids, triglycerides, and di- and monoglycerides can also serve as the carbon source

Particularly preferred nitrogen sources are urea, nitrate, nitrite, soy protein, amino acids, protein, com steep liquor, yeast extract, animal by-products, inorganic ammonium salt, more preferably ammonium salts of sulfate, hydroxide, and most preferably ammonium hydroxide. Other limiting nutrient sources include carbon sources (as defined above), phosphate sources, vitamin sources (such as vitamin B₁₂ sources, pantothenate sources, thiamine sources), and trace metal sources (such as zinc sources, copper sources, cobalt sources, nickel sources, iron sources, manganese sources, molybdenum sources), and major metal sources (such as magnesium sources, calcium sources,, sodium sources, potassium sources, and silica sources, etc.). Trace metal sources and major metal sources can include sulfate and chloride salts of these metals (for example but not limited to MgSO₄•7H₂O; MnCl₂•4H₂O; ZnSO₄•7H₂O; CoCl₂•6H₂O; Na₂MoO₄•2H₂O; CuSO₄•5H₂O; NiSO₄•6H₂O; FeSO₄•7H₂O; CaCl₂; K₂SO₄; KCl; and Na₂SO₄).

When ammonium is used as a nitrogen source, the fermentation medium becomes acidic if it is not controlled by base addition or buffers. When ammonium hydroxide is used as the primary nitrogen source, it can also be used to provide a pH control. The microorganisms of the order Thraustochytriales, in particular Thraustochytriales of the genus *Thrausiochytrium* and *Schizochytrium,* will grow over a wide pH range, *e.g*., from about pH 5 to about pH 11. A proper pH range for fermentation of a particular microorganism is within the knowledge of one skilled in the art.

Processes for growing microorganisms can also include a production stage. In this stage, the primary use of the substrate by the microorganisms is not increasing the biomass density but rather using the substrate to produce lipids. It should be appreciated that lipids are also produced by the microorganisms during the biomass density increasing stage; however, as stated above, the primary goal in the biomass density increasing stage is to increase the biomass density. Typically, during the production stage the addition of the limiting nutrient substrate is reduced or preferably stopped.

It was previously generally believed that the presence of dissolved oxygen (DO) in the fermentation medium is crucial in the production of polyunsaturated compounds, including omega-3 and/or omega-6 polyunsaturated fatty acids, by eukaryotic microorganisms. Thus, a relatively large amount of dissolved oxygen in the fermentation medium was generally believed to be preferred. Surprisingly and unexpectedly, however, the present inventors have found that the production rate of lipids is increased dramatically when the dissolved oxygen level during the production stage is reduced. Thus, while the dissolved oxygen level in the fermentation medium during the biomass density increasing stage is preferably at least about 8% of saturation, and preferably at least about 4% of saturation, during the production stage the dissolved oxygen in the fermentation medium is reduced to about 3% of saturation or less, preferably about 1% of saturation or less, and more preferably about 0% of saturation. At the beginning of the fermentation the DO can be at or near saturation and as the microbes grow it is allowed to drift down to these low DO setpoints. In one particular embodiment the amount of dissolved oxygen level in the fermentation medium is varied during the fermentation process. For example, for a fermentation process with total fermentation time of from about 90 hours to about 100 hours, the dissolved oxygen level in the fermentation medium is maintained at about 8% during the first 24 hours, about 4% from about 24^{th} hour to about 40^{th} hour, and about 0.5% or less from about 40^{th} hour to the end of the fermentation process.

The amount of dissolved oxygen present in the fermentation medium can be controlled by controlling the amount of oxygen in the head-space of the fermentor, or preferably by controlling the speed at which the fermentation medium is agitated (or stirred). For example, a high agitation (or stirring) rate results in a relatively higher amount of dissolved oxygen in the fermentation medium than a low agitation rate. For example, in a fermentor of about 14,000 gallon capacity the agitation rate is set at from about 50 rpm to about 70 rpm during the first 12 hours, from about 55 rpm to about 80 rpm during about 12^{th} hour to about 18^{th} hour and from about 70 rpm to about 90 rpm from about 18^{th} hour to the end of the fermentation process to achieve the dissolved oxygen level discussed above for a total fermentation process time of from about 90 hours to about 100 hours. A particular range of agitation speeds needed to achieve a particular amount of dissolved oxygen in the fermentation medium can be readily determined by one of ordinary skill in the art.

A preferred temperature for processes of the present invention is at least about 20°C, more preferably at least about 25°C, and most preferably at least about 30°C. It should be appreciated that cold water can retain a higher amount of dissolved oxygen than warm water. Thus, a higher fermentation medium temperature has the additional benefit of reducing the amount of dissolved oxygen, which is particularly desired as described above.

Certain microorganisms may require a certain amount of saline minerals in the fermentation medium. These saline minerals, especially chloride ions, can cause corrosion of the fermentor and other downstream processing equipment. To prevent or reduce these undesired effects due to a relatively large amount of chloride ions present in the fermentation medium, processes of the present invention can also include using non-chloride containing sodium salts, preferably sodium sulfate, in the fermentation medium as a source of sodium. More particularly, a significant portion of the sodium requirements of the fermentation is supplied as non-chloride containing sodium salts. For example, less than about 75% of the sodium in the fermentation medium is supplied as sodium chloride, more preferably less than about 50% and more preferably less than about 25%. The microorganisms of the present invention can be grown at chloride concentrations of less than about 3 g/L, more preferably less than about 500 mg/L, more preferably less than about 250 mg/L and more preferably between about 60 mg/L and about 120 mg/L.

Non-chloride containing sodium salts can include soda ash (a mixture of sodium carbonate and sodium oxide), sodium carbonate, sodium bicarbonate, sodium sulfate and mixtures thereof, and preferably include sodium sulfate. Soda ash, sodium carbonate and sodium bicarbonate tend to increase the pH of the fermentation medium, thus requiring control steps to maintain the proper pH of the medium. The concentration of sodium sulfate is effective to meet the salinity requirements of the microorganisms, preferably the sodium concentration is (expressed as g/L of Na) at least about 1 g/L, more preferably in the range of from about 1 g/L to about 50 g/L and more preferably in the range of from about 2 g/L to about 25 g/L.

Various fermentation parameters for inoculating, growing and recovering microorganisms are discussed in detail in U.S. Patent No. 5,130,242. Any currently known isolation methods can be used to isolate microorganisms from the fermentation medium, including centrifugation, filtration, ultrafiltration, decantation, and solvent evaporation. It has been found by the present inventors that because of such a high biomass density resulting from processes of the present invention, when a centrifuge is used to recover the microorganisms it is preferred to dilute the fermentation medium by adding water, which reduces the biomass density, thereby allowing more effective separation of microorganisms from the fermentation medium.

The very high biomass densities achieved in the present invention also facilitate "solventless" processes for recovery of microbial lipids. Preferred processes for lysing the cells in the fermentor are described in U.S. Provisional Patent Application Serial No. 60/177,125 entitled "SOLVENTLESS EXTRACTION PROCESS" filed January 19, 2000, U.S. Patent Application Serial No. 09/766,500 entitled "SOLVENTLESS EXTRACTION PROCESS" filed January 19, 2001, (now U.S. Patent No. 6,750,048) and PCT Patent Publication No. WO01/53512 entitled "SOLVENTLESS EXTRACTION PROCESS" filed January 19, 2001. Preferred processes for recovering the lipids once the cells are permeabilized, broken or lysed in the fermentor (which enables the lipid emulsion to be broken, and the lipid-rich fraction to be recovered) include the deoiling process outlined in WO 96/05278. In this process a water soluble compound, e.g., alcohol or acetone, is added to the oil/water emulsion to break the emulsion and the resulting mixture is separated by gravity separation, e.g., centrifugation. This process can also be modified to use other agents (water and/or lipid soluble) to break the emulsion.

Alternatively, the microorganisms are recovered in a dry form from the fermentation medium by evaporating water from the fermentation medium, *for example,* by contacting the fermentation medium directly (*i.e.,* without pre-concentration, for example, by centrifugation) with a dryer such as a drum-dryer apparatus, *i.e*., a direct drum-dryer recovery process. When using the direct drum-dryer recovery process to isolate microorganisms, typically a steam-heated drum-dryer is employed. In addition when using the direct drum-dryer recovery process, the biomass density of the fermentation medium is preferably at least about 130 g/L, more preferably at least about 150 g/L. and most preferably at least about 180 g/L. This high biomass density is generally desired for the direct drum-dryer recovery process because at a lower biomass density, the fermentation medium comprises a sufficient amount of water to cool the drum significantly, thus resulting in incomplete drying of microorganisms. Other methods of drying cells, including spray drying, are well known to one of ordinary skill in the art.

Processes of the present invention provide an average lipid production rate of at least 0.5 g/L/hr, preferably at least about 0.7 g/L/hr, more preferably at least about 0.9 g/L/hr, and most preferably at least about 1.0 g/L/hr. Moreover, lipids produced by processes of the present invention contain polyunsaturated lipids in the amount greater than about 15%, preferably greater than about 20%, more preferably greater than about 25%, still more preferably greater than about 30%, and most preferable greater than about 35%. Lipids can be recovered from either dried microorganisms or from the microorganisms in the fermentation medium. Generally, at least 20% of the lipids produced by the microorganisms in the processes of the present invention are omega-3 and/or omega-6 polyunsaturated fatty acids, preferably at least about 30% of the lipids are omega-3 and/or omega-6 polyunsaturated fatty acids, more preferably at least about 40% of the lipids are omega-3 and/or omega-6 polyunsaturated fatty acids, and most preferably at least about 50% of the lipids are omega-3 and/or omega-6 polyunsaturated fatty acids. Alternatively, processes of the present invention provides an average omega-3 fatty acid (e.g., DHA) production rate of at least 0.2 g of omega-3 fatty acid (e.g., DHA)/L/hr, preferably at least about 0.3 g of omega-3 fatty acid (e.g., DHA)/L/hr, more preferably at least about 0.4 g of omega-3 fatty acid (e.g., DHA)/L/hr, and most preferably at least about 0.5 g of omega-3 fatty acid (e.g., DHA)/L/hr. Alternatively, processes provide an average omega-6 fatty acid (e.g., DPAn-6) production rate of at least about 0.07 g of omega-6 fatty acid (e.g., DPAn-6)/L/hr, preferably at least about 0.1 g of omega-6 fatty acid (e.g., DPAn-6)/L/hr, more preferably at least about 0.13 g of omega-6 fatty acid (e.g., DPAn-6)L/hr, and most preferably at least about 0.17 g of omega-6 fatty acid (e.g., DPAn-6)/L/hr. Still alternatively, at least 25% of the lipid is DHA (based on total fatty acid methyl ester), preferably at least about 30%, more preferably at least about 35%, and most preferably at least about 40%.

Microorganisms, lipids extracted therefrom, the biomass remaining after lipid extraction or combinations thereof can be used directly as a food ingredient, such as an ingredient in beverages, sauces, dairy based foods (such as milk, yogurt, cheese and ice-cream) and baked goods; nutritional supplement (in capsule or tablet forms); feed or feed supplement for any animal whose meat or products are consumed by humans; food supplement, including baby food and infant formula; and pharmaceuticals (in direct or adjunct therapy application). The term "animal" means any organism belonging to the kingdom Animalia and includes, without limitation, any animal from which poultry meat, seafood, beef, pork or lamb is derived. Seafood is derived from, without limitation, fish, shrimp and shellfish. The term "products" includes any product other than meat derived from such animals, including, without limitation, eggs, milk or other products. When fed to such animals, polyunsaturated lipids can be incorporated into the flesh, milk, eggs or other products of such animals to increase their content of these lipids.

### EXAMPLES

The strain of *Schizochytrium* used in these examples produces two primary polyenoic acids, DHAn-3 and DPAn-6 in the ratio of generally about 3:1, and small amounts of other polyenoic acids, such as EPA and C20:3, under a wide variety of fermentation conditions. Thus, while the following examples only list the amount of DHA, one can readily calculate the amount of DPA(n-6) produced by using the above-disclosed ratio.

### Example 1

This example illustrates the effect of oxygen content in a fermentation medium on lipid productivity.

Fermentation results of *Schizochytrium* ATCC No. 20888 at various levels of dissolved oxygen content were measured. The results are shown in Figure 1, where RCS is residual concentration of sugar, and DCW is dry-cell weight.

### Example 2

This example also illustrates the effect of low oxygen content in the fermentation medium on DHA content (% dry weight) of the final biomass product.

A "scale-down" type experiment was conducted in 250 mL Erlenmeyer flasks to mimic the effect of low oxygen content on the DHA content in *Schizochytrium* sp. cells cultured in large-scale fermentors. *Schizochytrium* sp (ATCC 20888) was cultured in 04-4 medium. This culture media consisted of the following on a per liter basis dissolved in deionized water: Na₂SO₄ 12.61 g; MgSO₄•7H₂O 1.2g; KCl 0.25 g; CaCl₂ 0.05 g; monosodium glutamate 7.0 g; glucose 10 g; KH₂PO₄ 0.5 g; NaHCO₃ 0.1 g; yeast extract 0.1 g; vitamin mix 1.0 mL; PII metals 1.00 mL. PII metal mix contains (per liter): 6.0 g Na₂EDTA, 0.29 g FeCl₃•6H₂0, 6.84 g H₃BO₃, 0.86 g MnCl₂•4H₂O, 0.06 g ZnCl₂, 0.026 g CoCl₂•6H₂O, 0.052 g NiSO₄•H₂0, 0.002 CuSO₄•H₂O and 0.005 g Na₂MoO₄•2H₂O. Vitamin mix contains (per liter): 100 mg thiamine, 0.5 mg biotin and 0.5 mg cyanocobalamin. The pH of the culture media was adjusted to 7.0 and it was then filter sterilized.

The idea behind this scale-down experiment was to culture the cells in shake flasks with different volumes of culture media in the flasks - almost full flasks (e.g. 200 mL in a 250 mL shake flask) would not mix well on a shake table and therefore as the cells grew, low dissolved oxygen conditions would be generated. Therefore 4 treatments were established in the experiment, each conducted in duplicate: (1) 250 mL flasks filled with 50 mL culture medium; (2) 250 mL flasks filled with 100 mL culture medium; (3) 250 mL flasks filled with 150 mL culture medium; and (4) 250 mL shake flasks filled with 200 mL culture medium. Each of the eight flasks was inoculated with cells from a 48 hour old culture of *Schizochytrium* cultured in 04-4 medium under the conditions in treatment 1, and at 28°C and 220 rpm on a shaker table. All eight flasks for the experiment were placed on a shaker table (220 rpm) in a incubator (28°C) and cultured for 48 hours in the dark. At the end of the experiment, dissolved oxygen (DO) levels in each flask were measured with a YSI dissolved oxygen meter, (YSI Inc), pH of the culture medium was also determined, and the dry weight of cells and their fatty acid content was also measured. The results of the experiment are outlined in Table 1.

**Table 1. Results of scale-down experiment examining effect of low dissolved oxygen concentrations on the long chain highly unsaturated fatty acid content (DHA % dry weight) of Schizochytrium sp.**

| mL Medium | FAME (%TFA) | DHA (% dry wt) | Biomass (g/L) | Final PH | DO (% sat) |
|---|---|---|---|---|---|
| 50 | 16.5 | 7.4 | 4.2 | 7.4 | 31 |
| 100 | 17.0 | 6.5 | 3.9 | 7.2 | 29 |
| 150 | 22.4 | 9.2 | 2.7 | 7.0 | 11 |
| 200 | 35.9 | 14.5 | 1.8 | 6.9 | 3 |

The results indicate that the lipid content (as % FAME) and DHA content (% dry weight) were higher for cells cultured at low dissolved oxygen levels - the lower the dissolved oxygen level the higher the lipid and DHA content. This is unexpected because oxygen had been generally believed to be necessary to form desaturated (double) bonds. It is surprising that so much DHA was formed at low dissolved oxygen level, because DHA is one of the most unsaturated fatty acids. Although the biomass production decreases as the dissolved oxygen level is decreased, the DHA content is increased. Therefore, it is advantageous to have a growth phase with higher dissolved oxygen levels to maximize the formation of biomass and then lower the dissolved oxygen level to maximize long chain fatty acid production.

### Example 3

This example illustrates the reproducibility of processes of the present invention.

Microorganisms were produced using fermentors with a nominal working volume of 1,200 gallons (4,543 L). The resulting fermentation broth was concentrated and microorganisms were dried using a drum-dryer. Lipids from aliquots of the resulting microorganisms were extracted and purified to produce a refined, bleached, and deodorized oil. Approximately 3,000 ppm of d-1-α-tocopheryl acetate was added for nutritional supplementation purposes prior to analysis of the lipid.

Nine fermentations of *Schizochytrium* ATCC No. 20888 were run and the results are shown in Table 2. The dissolved oxygen level was about 8% during the first 24 hours and about 4% thereafter.

**Table 2. Fed-batch fermentation results for the production of DHA from Schizochytrium sp.**

| Entry | Age (Hrs) | Yield¹ (g/L) | DHA² (%) | FAME³ (%) | Productivity⁴ |
|---|---|---|---|---|---|
| 1 | 100.3 | 160.7 | 17.8 | 49.5 | 0.285 |
| 2 | 99.8 | 172.4 | 19.4 | 51.3 | 0.335 |
| 3 | 84.7 | 148.7 | 14.4 | 41.4 | 0.253 |
| 4 | 90.2 | 169.5 | 19.7 | 53.9 | 0.370 |
| 5 | 99.0 | 164.1 | 12.5 | 38.9 | 0.207 |
| 6 | 113.0 | 187.1 | 19.7 | 47.2 | 0.326 |
| 7 | 97.0 | 153.5 | 13.7 | 41.0 | 0.217 |
| 8 | 92.8 | 174.8 | 16.4 | 48.6 | 0.309 |
| Aver.⁵ | 97.1 | 166.4 | 16.7 | 46.5 | 0.288 |
| Std.⁶ | 8.4 | 12.3 | 2.9 | 5.4 | 0.058 |
| CV⁷(%) | 8.7 | 7.4 | 17.3 | 11.7 | 20.2 |

| | | | | | |
|---|---|---|---|---|---|
| 1. actual yield of biomass density. 2. DHA content as % cell dry weight. 3. total fatty acid content as % cell dry weight (measured as methyl esters). 4. (grams of DHA)/L/Hr. 5. average. 6. standard deviation 7. coefficients of variability. Coefficients of variability values below 5% indicate a process which has excellent reproducibility, values between 5% and 10% indicate a process which has good reproducibility and values between 10% and 20% indicate a process which has reasonable reproducibility. | | | | | |

Corn syrup was fed until the volume in the fermentor reached about 1,200 gallons (4,543 L), at which time the corn syrup addition was stopped. The fermentation process was stopped once the residual sugar concentration fell below 5 g/L. The typical age, from inoculation to final, was about 100 hours.

The fermentation broth, *i.e*., fermentation medium, was diluted with water using approximately a 2:1 ratio to reduce the ash content of the final product and help improve phase separation during the centrifugation step. The concentrated cell paste was heated to 160° F (about 71° C) and dried on a Blaw Knox double-drum dryer (42"x36"). Preferably, however, microorganisms are dried directly on a drum-dryer without prior centrifugation.

The analysis result of lipids extracted from aliquots of each entries in Table 2 is summarized in Table 3.

**Table 3. Analysis of the microbial biomass produced in the fed-batch fermentations outlined in Table. 2.**

| Entry | % DHA relative to FAME¹ | Total Lipid % by wt. |
|---|---|---|
| 1 | 36.0 | 72.3 |
| 2 | 37.8 | 70.3 |
| 3 | 34.8 | 61.5 |
| 4 | 36.5 | 74.8 |
| 5 | 32.1 | 52.8 |
| 6 | 41.7 | 67.7 |
| 7 | 33.4 | 49.9 |
| 8 | 33.7 | 61.4 |
| Average | 35.8 | 63.8 |
| Std. Deviation³ | 3.0 | 9.1 |
| CV⁴ (%) | 8.5 | 14.2 |

| | | |
|---|---|---|
| 1. see Table 2. 2. see discussion above. 3. standard deviation. 4. coefficients of variability. Coefficients of variability values below 5% indicates a process which has excellent reproducibility, values between 5% and 10% indicates a process which has good reproducibility and values between 10% and 20% indicates a process which has reasonable reproducibility. | | |

Unless otherwise stated, the fermentation medium used throughout the Examples section includes the following ingredients, where the first number indicates nominal target concentration and the number in parenthesis indicates acceptable range: sodium sulfate 12 g/L (11-13); KCl 0.5 g/L (0.45-0.55); MgSO₄•7H₂O 2 g/L (1.8-2.2); Hodag K-60 antifoam 0.35 g/L (0.3-0.4); K₂SO₄ 0.65 g/L (0.60-0.70); KH₂PO₄ 1 g/L (0.9-1.1); (NH₄)₂SO₄ 1 g/L (0.95-1.1); CaCl₂•2H₂O 0.17 g/L (0.15-0.19); 95 DE corn syrup (solids basis) 4.5 g/L (2-10); MnCl₂•4H₂O 3 mg/L (2.7-3.3); ZnSO₄•7H₂O 3 mg/L (2.7-3.3); CoCl₂•6H₂O 0.04 mg/L (0.035-0.045); Na₂MoO₄•2H₂O 0.04 mg/L (0-0.045); CuSO₄•5H₂O 2 mg/L (1.8-2.2); NiSO₄•6H₂O 2 mg/L (1.8-2.2); FeSO₄•7H₂O 10 mg/L (9-11); thiamine 9.5 mg/L (4-15); vitamin B₁₂ 0.15 mg/L (0.05-0.25) and Calcium Pantothenate 3.2 mg/L (1.3-5.1). In addition, 28% NH₄OH solution is used as the nitrogen source.

The ash content of the dried microorganisms is about 6% by weight.

### Example 4

This example illustrates the effect of reduced dissolved oxygen level in the fermentation medium on the productivity of microorganisms at the 14,000-gallon (52,996 L) scale.

Using the procedure described in Example 3, a 14,000-gallon (52,996 L) nominal volume fermentation was conducted using a wild-type strain *Schizochytrium,* which can be obtained using isolation processes disclosed in the above-mentioned U.S. Patent Nos. 5,340,594 and 5,340,742. The dissolved oxygen level in the fermentation medium was about 8% during the first 24 hours, about 4% from the 24^{th} hour to the 40^{th} hour and about 0.5% from the 40^{th} hour to the end of fermentation process. Results of this lower dissolved oxygen level in fermentation medium processes are shown in Table 4.

**Table 4. Results of 14,000-gallon (52,996 L) scale fed-batch fermentations of Schizochytrium at reduced dissolved oxygen concentrations.**

| Entry | Age (Hrs) | Yield (g/L) | %DHA | %FAME | %DHA rel. to FAME | DHA Productivity (g of DHA/L/hr) |
|---|---|---|---|---|---|---|
| 1 | 82.0 | 179.3 | 21.7 | 52.4 | 41.4 | 0.474 |
| 2 | 99.0 | 183.1 | 22.3 | 55.0 | 40.5 | 0.412 |
| 3 | 72.0 | 159.3 | - | - | 40.9 | - |
| 4 | 77.0 | 161.3 | - | - | 43.2 | - |
| 5 | 100.0 | 173.0 | 23.9 | 53.3 | 44.9 | 0.413 |
| 6 | 102.0 | 183.3 | 21.6 | 50.8 | 42.6 | 0.388 |
| 7 | 104.0 | 185.1 | 23.7 | 55.0 | 43.1 | 0.422 |
| 8 | 88.0 | 179.3 | 22.3 | 52.6 | 42.4 | 0.454 |
| 9 | 100.0 | 166.4 | 22.5 | 53.5 | 42.1 | 0.374 |
| 10 | 97.0 | 182.6 | 22.8 | 51.6 | 44.1 | 0.429 |
| 11 | 87.5 | 176.5 | 19.8 | 45.6 | 43.5 | 0.399 |
| 12 | 67.0 | 170.8 | 18.8 | 48.1 | 39.1 | 0.479 |
| 13 | 97.0 | 184.9 | 23.2 | 52.7 | 44.0 | 0.442 |
| 14 | 102.0 | 181.9 | 23.6 | 52.9 | 44.6 | 0.421 |
| 15 | 102.0 | 186.9 | 19.9 | 47.8 | 41.8 | 0.365 |
| 16 | 97.0 | 184.4 | 19.6 | 45.5 | 43.0 | 0.373 |
| 17 | 98.0 | 174.7 | 19.7 | 45.1 | 43.7 | 0.351 |
| 18 | 103.5 | 178.8 | 18:3 | 44.5 | 41.2 | 0.316 |
| 19 | 102.0 | 173.7 | 15.8 | 43.1 | 36.7 | 0.269 |
| 20 | 94.0 | 190.4 | 19.3 | 46.9 | 41.1 | 0.391 |
| 21 | 72.0 | 172.5 | 22.8 | 52.8 | 43.2 | 0.546 |
| 22 | 75.0 | 173.1 | 21.0 | 51.7 | 40.8 | 0.485 |
| 23 | 75.0 | 152.7 | 20.3 | 50.3 | 40.4 | 0.413 |
| 24 | 75.5 | 172.5 | 21.9 | 51.7 | 42.3 | 0.500 |
| 25 | 61.0 | 156.4 | 17.3 | 45.7 | 37.8 | 0.444 |
| 26 | 74.5 | 150.6 | 20.2 | 50.1 | 40.2 | 0.408 |
| 27 | 70.5 | 134.3 | 14.8 | 40.6 | 36.6 | 0.282 |
| 28 | 75.5 | 146.1 | 21.3 | 49.7 | 42.8 | 0.412 |
| 29 | 82.0 | 174.3 | 21.4 | 50.4 | 42.5 | 0.455 |
| 30 | 105.0 | 182.3 | 21.7 | 50.7 | 42.8 | 0.377 |
| 31 | 66.0 | 146.2 | 16.4 | 44.6 | 36.7 | 0.363 |
| | | | | | | |
| Avg | 87.2 | 171.5 | 20.6 | 49.5 | 41.6 | 0.409 |
| Std | 13.9 | 14.1 | 2.4 | 3.8 | 2.3 | 0.061 |
| CV | 16.0% | 8.2% | 11.6% | 7.7% | 5.5% | 15.0% |

### Example 5

This example illustrates the effect of reduced dissolved oxygen level in the fermentation medium on the productivity of microorganisms on a 41,000-gallon (155,202 L) scale.

The same procedures as Example 4 were employed except that the fermentation was conducted in a 41,000-gallon (155,202 L) fermentor. Culture media volumes were increased to maintain target compound concentrations at this scale. Results are shown in Table 5.

**Table 5. 41,000-gallon (155,202 L) scale fermentation of Schizochytrium**

| Entry | Age (Hrs) | Yield (g/L) | %DHA | %FAME | %DHA rel. to FAME | DHA Productivity (g of DHA/L/hr) |
|---|---|---|---|---|---|---|
| 1 | 75.0 | 116.1 | 17.3 | 46.1 | 37.4 | 0.268 |
| 2 | 99.0 | 159.3 | 17.4 | 47.0 | 37.1 | 0.280 |
| 3 | 103.0 | 152.6 | 16.0 | 47.2 | 33.8 | 0.237 |
| 4 | 68.0 | 136.8 | 17.9 | 45.9 | 39.1 | 0.360 |
| 5 | 84.0 | 142.0 | 17.5 | 47.0 | 37.2 | 0.296 |
| Avg | 85.8 | 141.4 | 17.2 | 46.6 | 36.9 | 0.288 |
| Std | 15.1 | 16.6 | 0.7 | 0.6 | 1.9 | 0.046 |
| CV | 17.5% | 11.8 | 4.2% | 1.3% | 5.2% | 15.8% |

### Example 6

This example illustrates the affect of extra nitrogen on the fermentation process of the present invention.

Four sets of 250-L scale fed-batch experiments were conducted using a procedure similar to Example 4. Two control experiments and two experiments containing extra ammonia (1.15x and 1.25x the normal amount) were conducted. Results are shown in Table 6.

**Table 6. Affects of extra ammonia on fermentation of Schizochytrium.**

| Age (hrs) | Yield (g/L) | Biomass Productivity | Conversion Efficiency | DHA Content | FAME Content | DHA Productivity |
|---|---|---|---|---|---|---|
| | | | | | | |
| Sugar target: 7 g/L, Base pH set point: 5.5, Acid pH set point: 7.3, 1.0X NH₃ | | | | | | |
| 48 | 178 | 3.71 g/L/hr | 51.5% | 10.7% | 37.8% | 0.40 g/L/hr |
| 60 | 185 | 3.08 g/L/hr | 46.9% | 16.3% | 47.2% | 0.50 g/L/hr |
| 72 | 205 | 2.85 g/L/hr | 45.2% | 17.4% | 47.4% | 0.50 g/L/hr |
| 84 | 219 | 2.61 g/L/hr | 43.8% | 17.1% | 45.5% | 0.45 g/L/hr |
| 90 | 221 | 2.46 g/L/hr | 44.1% | 18.4% | 48.9% | 0.45 g/L/hr |
| | | | | | | |

| Sugar target: 7 g/L, Base pH set point: 5.5, Acid pH set point: 7.3, 1.15X NH₃ | | | | | | |
|---|---|---|---|---|---|---|
| 48 | 171 | 3.56 g/L/hr | 55.6% | 12.0% | 36.3% | 0.43 g/L/hr |
| 60 | 197 | 3.28 g/L/hr | 54.6% | 9.4% | 38.4% | 0.31 g/L/hr |
| 72 | 191 | 2.65 g/L/hr | 52.8% | 9.4% | 40.0% | 0.25 g/L/hr |
| 84 | 190 | 2.26 g/L/hr | 52.5% | 10.0% | 42.5% | 0.23 g/L/hr |
| 90 | 189 | 2.10g/L/hr | 52.2% | 9.2% | 43.3% | 0.19 g/L/hr |
| | | | | | | |

| Sugar target: 7 g/L, Base pH set point: 5.5, Acid pH set point: 7.3, 1.25X NH₃ | | | | | | |
|---|---|---|---|---|---|---|
| 48 | 178 | 3.71 g/L/hr | 56.4% | 11.5% | 33.7% | 0.43 g/L/hr |
| 60 | 179 | 2.98 g/L/hr | 48.6% | 10.3% | 36.0% | 0.31 g/L/hr |
| 72 | 180 | 2.50 g/L/hr | 48.8% | 12.0% | 37.6% | 0.30 g/L/hr |
| 84 | 181 | 2.15 g/L/hr | 46.1% | 13.6% | 40.1% | 0.29 g/L/hr |
| 90 | 185 | 2.06 g/L/hr | 45.7% | 12.6% | 40.7% | 0.26 g/L/hr |
| | | | | | | |

| Sugar target: 7 g/L, Base pH set point: 5.5, Acid pH set point: 7.3, 1.0X NH₃ | | | | | | |
|---|---|---|---|---|---|---|
| 48 | 158 | 3.29 g/L/hr | 55.7% | 13.1% | 36.5% | 0.43 g/L/hr |
| 60 | 174 | 2.90 g/L/hr | 48.9% | 17,9% | 39.2% | 0.52 g/L/hr |
| 72 | 189 | 2.63 g/L/hr | 45.7% | 21.0% | 39.4% | 0.55 g/L/hr |
| 84 | 196 | 2.33 g/L/hr | 44.1% | 22.4% | 40.1% | 0.52 g/L/hr |
| 90 | 206 | 2.29 g/L/hr | 44.8% | 22.1% | 40.3% | 0.51 g/L/hr |

In general, extra nitrogen has a negative effect on fermentation performance, as significant reductions were observed in the DHA productivity for the two batches where extra ammonia was added. As shown on Table 6, the control batches resulted in final DHA levels of 18.4% and 22.1% of total cellular dry weight versus the 9.2% (1.15x ammonia) and 12.6% (1.25x ammonia) for extra nitrogen supplemented batches.

### Example 7

This example shows a kinetic profile of a fermentation process of the present invention. A 1000-gallon (3,785 L) scale fed-batch experiment was conducted using a procedure similar to Example 4. Kinetic profile of the fermentation process is shown in Table 7.

**Table 7. Kinetic Profile for a 1,000-gallon (3,785 L) scale Fed-Batch fermentation of Schizochytrium.**

| Age (hrs) | Yield (g/L) | Biomass Productivity | Conversion Efficiency | %DHA Content | %FAME Content | DHA Productivity |
|---|---|---|---|---|---|---|
| 24 | 118 | 4.92 g/L/hr | 78.2% | 7.4 | 18.8 | 0.36 g/L/hr |
| 30 | 138 | 4.60 g/L/hr | 60.3% | 10.6 | 30.9 | 0.49 g/L/hr |
| 36 | 138 | 3.83 g/L/hr | 46.6% | 11.6 | 36.5 | 0.44 g/L/hr |
| 42 | 175 | 4.17 g/L/hr | 49.8% | 13.4 | 41.7 | 0.56 g/L/hr |
| 48 | 178 | 3.71 g/L/hr | 45.1% | 18.7 | 52.8 | 0.69 g/L/hr |
| 48* | 164 | 3.42 g/L/hr | 41.5% | 15.3 | 33.1 | 0.52 g/L/hr |
| 54 | 196 | 3.63 g/L/hr | 45.7% | 16.6 | 51.2 | 0.60 g/L/hr |
| 60 | 190 | 3.17 g/L/hr | 41.7% | 16.9 | 33.9 | 0.54 g/L/hr |
| 72 | 189 | 2.62 g/L/hr | 39.1% | 15.6 | 31.8 | 0.41 g/L/hr |
| 84 | 195 | 2.32 g/L/hr | 38.5% | 16.4 | 32.7 | 0.38 g/L/hr |
| 90 | 200 | 2.22 g/L/hr | 39.0% | 18.8 | 33.3 | 0.42 g/L/hr |
| 90 | 171 | 1.90 g/L/hr | 33.3% | 22.2 | 61.6 | 0.42 g/L/hr ** |

| | | | | | | |
|---|---|---|---|---|---|---|
| * Two separate samples were analyzed at 48 hrs. ** This is for a washed dry-cell weights (DCW) sample. Other reported values are for unwashed samples. | | | | | | |

### Example 8

This example illustrates affect of the amount of carbon source on productivity.

Three different fermentation processes using the process of Example 4 were conducted using various amounts of carbon source. Results are shown on Table 8.

**Table 8. Fermentation results for various amounts of carbon source on fermentation of Schizochytrium.**

| Age (hrs) | Yield (g/L) | Carbon Charge | Conversion Efficiency | %DHA Content | %FAME Content | Productivity (g/L/hr) |
|---|---|---|---|---|---|---|
| 90 | 171 | 51.3% | 33.3% | 22.2 | 61.6 | 0.42 |
| 94 | 122 | 40.5% | 30.1% | 19.1 | 57.3 | 0.25 |
| 59 | 73 | 20.0% | 36.5% | 11.9 | 40.8 | 0.15 |

### Example 9

This example illustrates the effect of nutrient limitation on carbon conversion efficiency to biomass, lipid and most specifically DHA.

A continuous culture experiment to investigate the effect of nutrient limitation was performed by culturing *Schizochytrium* ATCC No. 20888 in a 2-liter volume Applikon fermentor in basal growth (ICM-2) medium consisting of the following compounds (nominal concentration): Group I ingredients: Na₂SO₄ (18.54 g/L), MgSO₄•7H₂0 (2.0 g/L) and KCL (0.572 g/L); Group II ingredients (each prepared separately): glucose (43.81 g/L), KH₂PO₄ (1.28 g/L), CaCl₂•2H₂0 (0.025 g/L) and (NH₄)₂SO₄ (6.538 g/L); Group III ingredients: Na₂EDTA (6.0 mg/L), FeCl₃•6H₂0 (0.29 mg/L), H₃BO₃ (6.84 mg/L), MnCl₂•4H₂0 (0.86 mg/L), ZnSO₄•7H₂0 (0.237 mg/L), CoCl₂•2H₂0 (0.026 mg/L), Na₂MoO₄•2H₂0 (0.005 mg/L), CuSO₄•5H₂0 (0.002 mg/L) and NiSO₄•6H₂0 (0.052 mg/L); and Group IV ingredients: thiamine HCl (0.2 mg/L), Vitamin B₁₂ (0.005 mg/L), Calcium pantothenate (0.2 mg/L). Groups I and II were autoclave sterilized, while Groups III and IV were filter sterilized prior to adding to the fermentor. The growth medium was then inoculated with *Schizochytrium* and grown under controlled conditions of 30°C, pH 5.5 and dissolved oxygen of 20% of saturation until maximum cell density was achieved.

A continuous operation mode was then established by simultaneously pumping sterile ICM-2 feed medium into the fermentor and removing the broth containing *Schizochytrium* cells at a flowrate sufficient to maintain a dilution rate of 0.06hr⁻¹, until a steady state is reached. To investigate the effect of nutrient limitation, the compound containing the specified required nutrient is lowered in the ICM-2 feed medium such that this nutrient is depleted in the outlet cell-containing broth, so that growth of the cells is limited by absence of the particular required nutrient. Once steady state operation was established for each condition, final broth dry biomass, residual glucose, and limiting nutrient concentrations, lipid content of the cell and DHA content of the cells were measured. The conversion efficiency of glucose to biomass was calculated by dividing the total glucose consumed by the total dried biomass formed, and expressed on a percentage basis.

The effects of limiting growth by each individual nutrient were studied by repeating this experiment for each individual nutrient listed in the following table. Final results are summarized in the following table:

**Table 9. Effect of nutrient limitation on the biomass yield, conversion efficiency (glucose -> biomass), lipid content and DHA content of Schizochytrium sp.**

| Limiting Nutrient | Biomass¹ (g/l) | Y_{x/s}² | RCS³ (g/l) | Lipid Content⁴ (%) | DHA Content⁵ (%) |
|---|---|---|---|---|---|
| Glucose | 18.7 | 46.8 | 0.0 | 19.8 | 7.3 |
| Nitrogen | 14.5 | 36.3 | 0.6 | 47.5 | 10.3 |
| Phosphate | 17.8 | 44.5 | 0.8 | 37.0 | 8.2 |
| Thiamine | 7.5 | 18.8 | 7.7 | 11.1 | 4.0 |
| Zinc | 16.0 | 40.0 | 1.3 | 27.8 | 7.2 |
| Copper | 14.0 | 35.0 | 10.4 | 13.8 | 5.3 |
| Cobalt | 14.5 | 36.3 | 0.0 | 22.2 | 6.9 |
| Nickel | 17.8 | 44.5 | 0.0 | 21.9 | 8.0 |
| Iron | 15.9 | 39.8 | 3.5 | 18.5 | 7.2 |
| Manganese | 12.5 | 31.3 | 3.4 | 26.1 | 8.0 |
| Magnesium | 13.9 | 34.8 | 5.3 | 18.7 | 6.4 |
| Calcium | 16.7 | 41.8 | 4.3 | 18.7 | 6.4 |
| Vitamin B12 | 19.6 | 49.0 | 0.0 | 17.5 | 6.3 |
| Molybdenum | 18.9 | 47.3 | 0.0 | 19.3 | 7.0 |
| Pantothenate | 19.2 | 48.0 | 0.0 | 20.4 | 6.7 |
| Sodium | 17.9 | 44.8 | 1.8 | 21.8 | 8.2 |
| Potassium | 13.0 | 32.5 | 8.8 | 14.1 | 5.3 |

| | | | | | |
|---|---|---|---|---|---|
| 1. concentration of dry biomass (grams/liter) 2. yield coefficient (% biomass produced/glucose consumed) 3. residual glucose concentration in broth (grams/liter) 4. lipid content of dry biomass (g lipid (as FAME)/g dry biomass) 5. DHA content of dry biomass (g DHA/g dry biomass) | | | | | |

It is clear from the table that nitrogen limitation resulted in the highest accumulation of DHA in the cells, followed by phosphate, sodium, nickel, manganese, glucose(carbon), zinc and iron. This information can be employed commercially by feeding one or more of these nutrients to a batch fermentation at a rate sufficient to limit cell growth. In the most preferred case, nitrogen is fed in a limiting manner to the batch fermentation to maximize the DHA content of the cells. Other nutrients (or mixtures thereof) can be fed in a limiting manner to maximize production of biomass or other valuable products. Other biologically required elements or nutrients that were not evaluated, such as sulfur, could also be employed as limiting nutrients in this fermentation control strategy.

Various preferred features and embodiments will now be described with reference to the following numbered paragraphs (paras).
1. A process for producing lipids containing polyenoic fatty acids from eukaryotic microorganisms capable of producing at least about 20% of their biomass as lipids comprising adding to a fermentation medium comprising said microorganisms a non-alcohol carbon source and a limiting nutrient source at a rate sufficient to increase the biomass density of said fermentation medium to at least about 100 g/L.
2. The process of para 1, wherein said process comprises a biomass density increasing stage and a production stage, wherein said biomass density increasing stage comprises adding said carbon source and said limiting nutrient source and said production stage comprises adding said carbon source with no or little additional said limiting nutrient source to induce nutrient limiting conditions which induces lipid production.
3. The process of para 2, wherein the amount of dissolved oxygen present in said fermentation medium during said production stage is lower than the amount of dissolved oxygen present in said fermentation medium during said biomass density increasing stage.
4. The process of para 3, wherein the amount of dissolved oxygen in said fermentation medium during said biomass density increasing stage is at least about 4%.
5. The process of para 4, wherein the amount of dissolved oxygen in said fermentation medium during said production stage is less than about 1%.
6. The process of para 1, wherein said non-alcohol carbon source comprises a carbohydrate.
7. The process of para 1, wherein said limiting nutrient source is selected from the group consisting of nitrogen sources, carbon_ sources, phosphate sources, vitamin sources (such as vitamin B₁₂ sources, pantothenate sources, thiamine sources), trace metal sources (such as zinc sources, copper sources, cobalt sources, nickel sources, iron sources, manganese sources, molybdenum sources), and major metal sources (such as magnesium sources, calcium sources, sodium sources, potassium sources), silica sources and mixtures thereof
8. The process of para 7, wherein said trace metal sources and major metal sources are selected from the group consisting of sulfate and chloride salts of these metals (such as MgSO₄•7H₂O; MnCl₂•4H₂O ; ZnSO₄•7H₂O; CoCl₂•6H₂O ; Na₂MoO₄•2H₂O ; CuSO₄•5H₂O ; NiSO₄•6H₂O ; FeSO₄•7H₂O ; CaCl₂; K₂SO₄; KCl; and Na₂SO₄) and mixtures thereof
9. The process of para 1, wherein said limiting nutrient source comprises a nitrogen source.
10. The process of para 9, wherein said nitrogen source comprises an inorganic ammonium salt.
11. The process of para 9, wherein said nitrogen source comprises ammonium hydroxide.
12. The process of para 10, wherein pH of the fermentation medium is controlled by said limiting nutrient source.
13. The process of para 1, wherein said fermentation medium is at a temperature of at least about 20°C.
14. The process of para 1, wherein said process produces lipids at an average rate of at least about 0.5 g/L/hr.
15. The process of para 14, wherein at least about 15% of said lipids are polyunsaturated lipids.
16. The process of para 14, wherein the total amount of omega-3 and omega-6 fatty acids is at least about 20% of said lipids.
17. The process of para 14, wherein at least about 25% of said lipids is docosahexaenoic acid.
18. The process of para 1, wherein said microorganisms are selected from the group consisting of algae, fungi (including yeast), protists, bacteria and mixtures thereof, wherein said microorganisms are capable of producing polyenoic fatty acids or other lipids which had been generally believed to require oxygen for their synthesis.
19. The process of para 18, wherein said microorganisms are capable of producing lipids at a fermentation medium oxygen level of about less than 3% of saturation.
20. Theprocessof para 18, wherein said microorganisms are grown in a fed-batch process.
21. The process of para 20 further comprising maintaining an oxygen level of less than about 3% of saturation in said fermentation medium during the production phase of said fermentation process.
22. The process of para 1, wherein said microorganisms are microalgae and algae-like microorganisms.
23. The process of para 1, wherein said microorganisms are Stramenopiles.
24. The process of para 1, wherein said microorganisms are of the order Thraustochytriales.
25. The process of para 1, wherein said microorganisms are selected from the group consisting of *Thraustochytrium, Schizochytrium,* and mixtures thereof.
26. A process for growing eukaryotic microorganisms capable of producing at least about 20% of their biomass as lipids, said process comprising adding to a fermentation medium comprising said microorganisms a carbon source and a limiting nutrient source at a rate sufficient to increase the biomass density of said fermentation medium to at least about 100 g/L, wherein said process produces at least an average rate of about 0.5 g/L/hr of lipids, arid wherein at least about 15% of the total lipids produced by said microorganisms is polyunsaturated lipids.
27. The process of para 26, wherein said process further comprises:
   (a) a biomass density increasing stage, wherein the dissolved oxygen level in the fermentation medium is at least about 4%; and
   (b) a high lipid production stage, wherein the dissolved oxygen level in the fermentation medium is less than about 1%.
28. The process of para 26, wherein said carbon source is a non-alcohol carbon source.
29. The process of para 26, wherein said limiting nutrient source is selected from the group consisting of nitrogen sources, carbon sources, phosphate sources, vitamin sources (such as vitamin B₁₂ sources, pantothenate sources, thiamine sources), trace metal sources (such as zinc sources, copper sources, cobalt sources, nickel sources, iron sources, manganese sources, molybdenum sources), and major metal sources (such as magnesium sources, calcium sources, sodium sources, potassium sources), and silica sources and mixtures thereof.
30. The process of para 29, wherein said trace metal sources and major metal sources are selected from the group consisting of sulfate and chloride salts of these metals (such as MgSO₄•7H₂O; MnCl₂•4H₂O; ZnSO₄•7H₂O; CoCl₂•6H₂O; Na₂MoO₄•2H₂O; CuSO₄•5H₂O; NiSO₄•6H₂O; FeSO₄•7H₂O; CaCl₂; K₂SO₄; KCl; and Na₂SO₄) and mixtures thereof.
31. The process of para 26, wherein said limiting nutrient source comprises a nitrogen source.
32. The process of para 31, wherein said nitrogen source comprises an inorganic ammonium salt.
33. The process of para 26, wherein said microorganisms are selected from the group consisting of algae, fungi (including yeast), protists, bacteria and mixtures thereof, wherein said microorganisms are capable of producing polyenoic fatty acids or other lipids which had been generally believed to requireoxygen for their synthesis.
34. The process of para 26, wherein said microorganisms are Stramenopiles.
35. The process of para 26, wherein said microorganisms are microalgae.
36. The process of para 26, wherein said microorganisms are of the order Thraustochytriales.
37. The process of para 26, wherein said microorganisms are selected from the group consisting of *Thraustochytrium, Schizochytrium,* and mixtures thereof.
38. The process of para 26, wherein the total amount of omega-3 and omega-6 fatty acids is at least about 20 % of said lipids.
39. The process of para 26, wherein at least about 25 % of said lipids is docosahexaenoic acid.
40. A process for growing eukaryotic microorganisms comprising adding to a fermentation medium comprising said microorganisms a carbon source and a limiting nutrient source at a rate sufficient to increase the biomass density of said fermentation medium to at least about 100 g/L, wherein said microorganisms are selected from the group consisting of algae, fungi (including yeast), protists, bacteria and mixtures thereof, and wherein said microorganisms are capable of producing at least about 20% of their biomass as lipids comprising some polyenoic fatty acids.
41. The process of para 40, wherein said process produces microbial lipids at an average rate of least about 0.5 g/L/hr.
42. The process of para 41, wherein greater than about 15% of said lipids are polyunsaturated lipids.
43. The process of para 41, wherein the total amount of omega-3 and omega-6 polyunsaturated fatty acids are at least about 20% of said lipids.
44. The process of para 41, wherein at least about 25% of said lipids is docosahexaenoic acid.
45. The process of para 40, wherein said process produces on average at least about 0.2 g/L/hr of docosahexaenoic acid.
46. The process of para 40, wherein said carbon source is not an alcohol.
47. The process of para 46, wherein said carbon source is a carbohydrate.
48. The process of para 40, wherein said limiting nutrient source is selected from the group consisting of nitrogen sources, carbon sources, phosphate sources, vitamin sources (such as vitamin B₁₂ sources, pantothenate sources, thiamine sources), trace metal sources (such as zinc sources, copper sources, cobalt sources, nickel sources, iron sources, manganese sources, molybdenum sources), and major metal sources (such as magnesium sources, calcium sources, sodium sources, potassium sources), silica sources, and mixtures thereof.
49. The process of para 48, wherein said trace metal sources and major metal sources are selected from the group consisting of sulfate and chloride salts of these metals (such as MgSO₄•7H₂O; MnCl₂•4H₂O; ZnSO₄•7H₂O; CoCl₂•6H₂O; Na₂MoO₄•2H₂O; CuSO₄•5H₂O; NiSO₄•6H₂O; FeSO₄•7H₂O; CaCl₂; K₂SO₄; KCl; and Na₂SO₄) and mixtures thereof.
50. The process of para 40, wherein said limiting nutrient sources comprises a nitrogen source.
51. The process of para 50, wherein said nitrogen source comprises an inorganic ammonium salt.
52. The process of para 40, wherein said nitrogen source comprises ammonium hydroxide.
53. The process of para 40, wherein said microorganisms are selected from the group consisting of algae, fungi (including yeast), protists, bacteria and mixtures thereof, wherein said microorganisms are capable of producing polyenoic fatty acids or other lipids which had been generally believed to require oxygen for their synthesis.
54. The process of para 40, wherein said microorganisms are microalgae and algae-like microorganisms.
55. The process of para 40, wherein said microorganisms are Stramenopiles.
56. A process for producing eukaryotic microbial lipids comprising:
   (a) growing eukaryotic microorganisms in a fermentation medium to increase the biomass density of said fermentation medium to at least about 100 g/L;
   (b) providing fermentation conditions sufficient to allow said microorganisms to produce said lipids; and
   (c) recovering said lipids,wherein greater than about 15% of said lipids are polyunsaturated lipids.
57. The process of para 56, wherein a dissolved oxygen level in said fermentation medium during said microorganism growing step is higher than the dissolved oxygen level in the fermentation medium during said lipid producing step.
58. The process of para 56, wherein the dissolved oxygen level in said fermentation medium during the microorganism growing step is at least about 4%.
59. The process of para 56, wherein the dissolved oxygen level in said fermentation medium during the lipid producing step is less than about 1%.
60. The process of para 56, wherein said microorganisms are selected from the group consisting of algae, fungi (including yeast), protists, bacteria and mixtures thereof, wherein said microorganisms are capable of producing polyenoic fatty acids or other lipids which had been generally believed to require oxygen for their synthesis.
61. The process of para 56, wherein said microorganisms are Stramenopiles.
62. The process of para 56, wherein said microorganisms are microalgae and algae-like microorganisms.
63. The process of para 56, wherein said microorganisms are of the order Thraustochytriales.
64. The process of para 56, wherein said microorganisms are selected from the group consisting of *Thraustochytrium, Schizochytrium,* and mixtures thereof.
65. The process of para 56, wherein the total amount of omega-3 and omega-6 fatty acids is at least about 20% of said microbial lipids.
66. The process of para 56, wherein at least about 25% of said microbial lipids is docosahexaenoic acid.
67. The process of para 56, wherein said process produces docosahexaenoic acid at an average rate of at least about 0.2 g/L/hr.
68. The process of para 56, wherein said lipid recovery step comprises:
   (d) removing water from said fermentation medium to provide dry microorganisms; and
   (e) isolating said lipids from said dry microorganisms.
69. The process of para 68, wherein said water removal step comprises contacting said fermentation medium directly on a drum-dryer without prior centrifugation.
70. The process of para 56, wherein said lipid recovery step comprises:
   (d) treating the fermentation broth to permeabilize, lyse or rupture the microbial cells; and
   (e) recovering the lipids from the fermentation broth by gravity separation, with or without the aid of anagent to aid in breaking the lipid/water emulsion.
71. The process of para 70, wherein said microbial cells are treated in step (d) in a fermentor or a similar vessel.
72. The process of para 70, wherein said gravity separation comprises centrifugation.
73. The process of para 56, wherein said microorganism growing step comprises adding a carbon source and a limiting nutrient source.
74. The process of para 73, wherein said carbon source is non-alcohol.
75. The process of para 74, wherein said carbon source comprises a carbohydrate.
76. The process of para 73, wherein said limiting nutrient source is selected from the group consisting of nitrogen sources, carbon_sources, phosphate sources, vitamin sources (such as vitamin B₁₂ sources, pantothenate sources, thiamine sources), trace metal sources (such as zinc sources, copper sources, cobalt sources, nickel sources, iron sources, manganese sources, molybdenum sources), and major metal sources (such as magnesium sources, calcium sources, sodium sources, potassium sources), silica sources, and mixtures thereof.
77. The process of para 76, wherein said trace metal sources and major metal sources are selected from the group consisting of sulfate and chloride salts of these metals (such as MgSO₄•7H₂O; MnCl₂•4H₂O; ZnSO₄•7H₂O; CoCl₂•6H₂O ; Na₂MoO₄•2H₂O; CuSO₄•5H₂O; NiSO₄•6H₂O ; FeSO₄•7H₂O ; CaCl₂; K₂SO₄; KCl; and Na₂SO₄) and mixtures thereof.
78. The process of para 73, wherein said limiting nutrient source comprises a nitrogen source.
79. The process of para 78, wherein said nitrogen source comprises an inorganic ammonium salt.
80. The process of para 78, wherein said nitrogen source comprises ammonium hydroxide.
81. A process for producing eukaryotic microbial lipids comprising:
   (a) adding a non-alcohol carbon source and a limiting nutrient source to a fermentation medium comprising said microorganisms;
   (b) providing conditions sufficient for said microorganisms to produce said microbial lipids; and
   (c) recovering said microbial lipids,
   wherein at least about 15% of said microbial lipids are polyunsaturated lipids.
82. The process of para 81, wherein the dissolved oxygen level in said fermentation medium during a biomass density increasing step is higher than the dissolved oxygen level in said fermentation medium during a lipid producing step.
83. The process of para 81, wherein the dissolved oxygen level in said fermentation medium during a biomass density increasing step is at least about 4%.
84. The process of para 81, wherein the dissolved oxygen level in said fermentation medium during a lipid producing step is about 1% or less.
85. The process of para 81, wherein said microorganisms are selected from the group consisting of algae, fungi (including yeast), protists, bacteria and mixtures thereof.
86. The process of para 81, wherein said microorganisms are selected from the group consisting of algae, fungi (including yeast), protists, bacteria and mixtures thereof, wherein said microorganisms are capable of producing polyenoic fatty acids or other lipids which had been generally believed to require oxygen for their synthesis.
87. The process of para 81, wherein said microorganisms are Stramenopiles.
88. The process of para 81, wherein said microorganisms are microalgae and algae-like microorganisms.
89. The process of para 81, wherein said microorganisms are of the order Thraustochytriales.
90. The process of para 81, wherein said microorganisms are selected from the group consisting of *Thraustochytrium, Schizochytrium,* and mixtures thereof.
91. The process of para 81, wherein said process produces said microbial lipids at an average rate of at least about 0.5 g/L/hr.
92. The process of para 91, wherein the total amount ofomega-3 and omega-6 fatty acids is at least about 20% of said microbial lipids.
93. The process of para 81, wherein at least about 25% of said lipids is docosahexaenoic acid.
94. The process of para 81, wherein said process produces docosahexaenoic acid at an average rate of at least about 0.2 g/L/hr.
95. The process of para 58, wherein said lipid recovery step comprises:
   (d) removing water from said fermentation medium to provide dry microorganisms; and
   (e) isolating said lipids from said dry microorganisms.
96. The process of para 95, wherein said water removal step comprises evaporating water from said fermentation medium without prior centrifugation.
97. The process of para 81, wherein said lipid recovery step comprises:
   (d) treating the fermentation broth to permeabilize, lyse or rupture the microbial cells; and
   (e) recovering the lipids from the fermentation broth by gravity separation, with or without the aid of an agent to aid in breaking the lipid/water emulsion.
98. The process of para 97, wherein said microbial cells are treated in step (d) in a fermentor or a similar vessel.
99. The process of para 97, wherein said gravity separation comprises centrifugation.
100. The process of para 81, wherein said non-alcohol carbon source comprises a carbohydrate.
101. Theprocessof para 81, wherein said limiting nutrient source is selected from the group consisting of nitrogen sources, carbon sources, phosphate sources, vitamin sources (such as vitamin B₁₂ sources, pantotheriate sources, thiamine sources), trace metal sources (such as zinc sources, copper sources, cobalt sources, nickel sources, iron sources, manganese sources, molybdenum sources), and major metal sources (such as magnesium sources, calcium sources, sodium sources, potassium sources), silica sources, and mixtures thereof.
102. The process of para 101, wherein said trace metal sources and major metal sources are selected from the group consisting of sulfate and chloride salts of these metals (such as MgSO₄•7H₂O; MnCl₂•4H₂O; ZnSO₄•7H₂O; CoCl₂•6H₂O; Na₂MoO₄•2H₂O; CuSO₄•5H₂O ; NiSO₄•6H₂O; FeSO₄•7H₂O; CaCl₂; K₂SO₄; KCl; and Na₂SO₄) and mixtures thereof.
103. The process of para 81, wherein said limiting nutrient source comprises a nitrogen source.
104. The process of para 103, wherein said nitrogen source comprises an inorganic ammonium salt.
105. The process of para 103, wherein said nitrogen source comprises ammonium hydroxide.
106. A process for producing eukaryotic microbial lipids comprising:
   (a) adding a carbon source and a limiting nutrient source to a fermentation medium comprising eukaryotic microorganisms and providing conditions sufficient to maintain a dissolved oxygen level of at least about 4% in said fermentation medium to produce a biomass density of at least about 100mL;
   (b) providing conditions sufficient to maintain a dissolved oxygen level of about 1% or less in said fermentation medium and providing conditions sufficient to allow said microorganisms to produce said lipids; and
   (c) recovering said microbial lipids,
   wherein at least about 15% of said microbial lipids are polyunsaturated lipids.
107. The process of para 106, wherein said microorganisms are selected from the group consisting of algae, fungi (including yeast), protists, bacteria and mixtures thereof.
108. The process of para 106, wherein said microorganisms are selected from the group consisting of algae, fungi (including yeast), protists, bacteria and mixtures thereof, wherein said microorganisms are capable of producing polyenoic fatty acids or other lipids which had been generally believed to require oxygen for their synthesis.
109. The process of para 106, wherein said microorganisms are Stramenopiles.
110. The process of para 106, wherein said microorganisms are microalgae and algae-like microorganisms.
111. The process of para 106, wherein said microorganisms are of the order Thraustochytriales.
112. The process of para 106, wherein said microorganisms are selected from the group consisting of *Thraustochytrium, Schizochytrium,* and mixtures thereof.
113. The process of para 106, wherein at least about 20% of said microbial lipids is omega-3 and/or omega-6 fatty acids.
114. The process of para 106, wherein at least about 25% of said microbial lipids is docosahexaenoic acid.
115. The process of para 106, wherein said process produces docosahexaenoic acid at an average rate of at least about 0.2 g/L/hr.
116. The process of para 106, wherein the lipid recovery step comprises:
   (d) removing water from said fermentation medium to provide dry microorganisms; and
   (e) isolating said lipids from said dry microorganisms.
117. The process of para 116, wherein the water removal step comprises evaporating the water directly from said fermentation medium without prior centrifugation.
118. The process of para 106, wherein the lipid recovery step comprises:
   (d) treating the fermentation broth to permeabilize, lyse or rupture the microbial cells; and
   (e) recovering the lipids from the fermentation broth by gravity separation, with or without the aid of a agent to aid in breaking the lipid/water emulsion.
119. The process of para 106, wherein said microbial cells are treated in step (d) in a fermentor or a similar vessel.
120. The process of para 118, wherein said gravity separation comprises centrifugation.
121. A method for enriching the polyenoic fatty acid content of a microorganism comprising fermenting said microorganism in a growth medium having a level of dissolved oxygen of less than 10%.
122. The process of para 121, wherein said level of dissolved oxygen is less than 5 percent.
123. The process of para 121, wherein said level of dissolved oxygen is less than 1 percent.
124. The process of para 121, wherein during an initial growth phase, the level of dissolved oxygen is greater than about 10 percent and during a subsequent production phase the level of dissolved oxygen is less than 10 percent.
125. The process of para 121, wherein said microorganism contains polyketide synthase genes.
126. A heterotrophic process for producing products and microorganisms comprising:
   a. culturing said microorganisms in a growth medium wherein said microorganisms contain polyketide synthase genes; and
   b. maintaining the level of dissolved oxygen at less than about 10 percent.
127. The process of para 126, wherein said level of dissolved oxygen is less than about 5 percent.
128. The process of para 126, wherein said level of dissolved oxygen is less than about 1 percent.
129. Thepracess of para 126, wherein said polyketide synthase genes occur naturally in said microorganisms.
130. The process of para 126, wherein said polyketide synthase genes are genetically introduced into the said microorganisms.
131. The process of para 126, wherein during an initial growth phase, the level of dissolved oxygen is greater than about 10 percent and during a subsequent production phase the level of dissolved oxygen is less than about 10 percent.

## Claims

1. A process for growing eukaryotic microorganisms of the order of Thraustochytriales comprising adding to a fermentation medium comprising said microorganisms a carbon source and a limiting nutrient source at a rate sufficient to increase the biomass density of said fermentation medium to at least 100 g/L on a dry cell weight basis, wherein said microorganisms are capable of producing at least 20% of their biomass as lipids comprising some polyenoic fatty acids.

2. The process of Claim 1, wherein said process produces microbial lipids at an average rate of least 0.5 g/L/hr.

3. The process of Claim 2, wherein greater than 15% of said lipids are polyunsaturated lipids or wherein the total amount of omega-3 and omega-6 polyunsaturated fatty acids are at least 20% of said lipids.

4. The process of any one of claims 1 to 3, wherein the lipids comprise omega-3 and/or omega-6 fatty acids, optionally docosahexaenoic acid (DHA), docosapentaenoic acid (DPA), eicosapentaenoic acid (EPA), or arachidonic acid (ARA).

5. The process of Claim 2, wherein at least 25% of said lipids is docosahexaenoic acid.

6. The process according to any preceding claim, wherein said process produces on average at least 0.2 g/L/hr of docosahexaenoic acid.

7. The process according to any preceding claim, wherein said carbon source is not an alcohol, optionally, wherein said carbon source is a carbohydrate.

8. The process according to any preceding claim, wherein said limiting nutrient source is selected from the group consisting of (i) nitrogen sources, (ii) carbon sources, (iii) phosphate sources, (iv) vitamin sources, such as vitamin B i2 sources, pantothenate sources, thiamine sources, (v) trace metal sources, such as zinc sources, copper sources, cobalt sources, nickel sources, iron sources, manganese sources, molybdenum sources, and (vi) major metal sources, such as magnesium sources, calcium sources, sodium sources, potassium sources, (vii) silica sources, and mixtures thereof.

9. The process of Claim 8, wherein said trace metal sources and major metal sources are selected from the group consisting of sulfate and chloride salts of these metals (such as MgSO₄•7H₂O; MnCl₂•4H₂O; ZnSO₄•7H₂O; CoCl₂•6H₂O; Na₂MoO₄•2H₂O; CuSO₄•5H₂O; NiSO₄•6H₂O; FeSO₄•7H₂O; CaCl₂; K₂SO₄; KCl; and Na₂SO₄) and mixtures thereof.

10. The process according to any preceding claim, wherein said limiting nutrient sources comprises a nitrogen source, for instance an inorganic ammonium salt.

11. The process of Claim 10, wherein said nitrogen source comprises ammonium hydroxide.

12. The process according to any preceding claim, wherein said microorganisms are of the genus *Thraustochytrium* or *Schyzochytrium.*

13. The process of any preceding claim wherein at least 20% of the lipids produced by the microorganisms are omega-3 and/or omega-6 polyunsaturated fatty acids, for instance wherein at least 30% of the lipids produced by the microorganisms are omega-3 and/or omega-6 polyunsaturated fatty acids, for instance wherein at least 40% of the lipids produced by the microorganisms are omega-3 and/or omega-6 polyunsaturated fatty acids, for instance wherein at least 50% of the lipids produced by the microorganisms are omega-3 and/or omega-6 polyunsaturated fatty acids.

14. The process of any preceding claim, further comprising recovering lipids from the microorganisms.

15. The process of any preceding claim, further comprising using the microorganisms, lipids extracted therefrom, biomass remaining after lipid extraction or combinations thereof as a feed or feed supplement for an animal whose meat or products are consumed by humans, for instance an animal from which poultry meat, seafood, beef, pork or lamb is derived.

## Patentansprüche

1. Verfahren zur Kultivierung eukaryontischer Mikroorganismen der Ordnung Thraustochytriales, umfassend Zugeben einer Kohlenstoffquelle und einer limitierenden Nährstoffquelle zu einem die Mikroorganismen umfassenden Fermentationsmedium mit einer hinreichenden Geschwindigkeit, so dass die Biomassendichte des Fermentationsmediums auf wenigstens 100 g/l auf Zelltrockengewichtsbasis erhöht wird, wobei die Mikroorganismen in der Lage sind, wenigstens 20% ihrer Biomasse als Lipide, die einige Polyenfettsäuren umfassen, zu produzieren.

2. Verfahren nach Anspruch 1, wobei bei dem Verfahren mikrobielle Lipide mit einer Durchschnittsgeschwindigkeit von wenigstens 0,5 g/l pro Std. produziert werden.

3. Verfahren nach Anspruch 2, wobei es sich bei mehr als 15% der Lipide um mehrfach ungesättigte Lipide handelt oder wobei die Gesamtmenge an mehrfach ungesättigten Omega-3- und Omega-6-Fettsäuren wenigstens 20% der Lipide beträgt.

4. Verfahren nach einem der Ansprüche 1 bis 3, wobei die Lipide Omega-3- und/oder Omega-6-Fettsäuren, gegebenenfalls Docosahexaensäure (DHA), Docosapentaensäure (DPA), Eicosapentaensäure(EPA) oder Arachidonsäure (ARA) umfassen.

5. Verfahren nach Anspruch 2, wobei es sich bei wenigstens 25% der Lipide um Docosahexaensäure handelt.

6. Verfahren gemäß einem vorhergehenden Anspruch, wobei mit dem Verfahren im Durchschnitt wenigstens 0,2 g/l pro Std. Docosahexaensäure produziert werden.

7. Verfahren gemäß einem vorhergehenden Anspruch, wobei es sich bei der Kohlenstoffquelle nicht um einen Alkohol handelt, gegebenenfalls wobei es sich bei der Kohlenstoffquelle um ein Kohlenhydrat handelt.

8. Verfahren gemäß einem vorhergehenden Anspruch, wobei die limitierende Nährstoffquelle aus der aus (i) Stickstoffquellen, (ii) Kohlenstoffquellen, (iii) Phosphatquellen, (iv) Vitaminquellen wie Vitamin-B-12-Quellen, Pantothenatquellen, Thiaminquellen, (v) Spurenmetallquellen wie Zinkquellen, Kupferquellen, Cobaltquellen, Nickelquellen, Eisenquellen, Manganquellen, Molybdänquellen und (vi) Hauptmetallquellen wie Magnesiumquellen, Calciumquellen, Natriumquellen, Kaliumquellen, (vii) Siliciumdioxidquellen und Gemischen davon bestehenden Gruppe ausgewählt ist.

9. Verfahren nach Anspruch 8, wobei die Spurenmetallquellen und Hauptmetallquellen aus der aus Sulfat- und Chloridsalzen dieser Metalle (wie MgSO₄•7H₂O; MnCl₂•4H₂O; ZnSO₄•7H₂O; CoCl₂•6H₂O; Na₂MoO₄•2H₂O; CuSO₄•5H₂O; NiSO₄•6H₂O; FeSO₄•7H₂O; CaCl₂; K₂SO₄; KCl; und Na₂SO₄) und Gemischen davon bestehenden Gruppe ausgewählt sind.

10. Verfahren gemäß einem vorhergehenden Anspruch, wobei die limitierende Nährstoffquelle eine Stickstoffquelle, beispielsweise ein anorganisches Ammoniumsalz, umfasst.

11. Verfahren nach Anspruch 10, wobei die Stickstoffquelle Ammoniumhydroxid umfasst.

12. Verfahren gemäß einem vorhergehenden Anspruch, wobei die Mikroorganismen zur Gattung *Thraustochytrium* oder *Schyzochytrium* gehören.

13. Verfahren nach einem vorhergehenden Anspruch, wobei es sich bei wenigstens 20% der von den Mikroorganismen produzierten Lipide um mehrfach ungesättigte Omega-3- und/oder Omega-6-Fettsäuren handelt, beispielsweise wobei es sich bei wenigstens 30% der von den Mikroorganismen produzierten Lipide um mehrfach ungesättigte Omega-3- und/oder Omega-6-Fettsäuren handelt, beispielsweise wobei es sich bei wenigstens 40% der von den Mikroorganismen produzierten Lipide um mehrfach ungesättigte Omega-3- und/oder Omega-6-Fettsäuren handelt, beispielsweise wobei es sich bei wenigstens 50% der von den Mikroorganismen produzierten Lipide um mehrfach ungesättigte Omega-3- und/oder Omega-6-Fettsäuren handelt.

14. Verfahren nach einem vorhergehenden Anspruch, ferner umfassend Gewinnen von Lipiden aus den Mikroorganismen.

15. Verfahren nach einem vorhergehenden Anspruch, ferner umfassend Verwenden der Mikroorganismen, davon extrahierter Lipide, nach Lipidextraktion verbleibender Biomasse oder von Kombinationen davon als Futter oder Futterzusatz für ein Tier, dessen Fleisch oder Produkte vom Menschen konsumiert werden, beispielsweise ein Tier, von dem Geflügelfleisch, Meeresfrucht, Rind-, Schweine- oder Lammfleisch gewonnen wird.

## Revendications

1. Procédé de culture de microorganismes eucaryotes de l'ordre des Thraustochytriales, comprenant l'addition à un milieu de fermentation comprenant lesdits microorganismes, d'une source de carbone et d'une source nutritive limitante à une vitesse suffisante pour augmenter la densité de biomasse dudit milieu de fermentation jusqu'à au moins 100 g/l sur une base pondéral de cellules sèches, où lesdits microorganismes sont capables de produire au moins 20% de leur biomasse sous forme de lipides comprenant certains acides gras polyénoïques.

2. Procédé selon la revendication 1, où ledit procédé produit des lipides microbiens à une vitesse moyenne d'au moins 0,5 g/l/h.

3. Procédé selon la revendication 2, où plus de 15% desdits lipides sont des lipides polyinsaturés ou où la quantité totale d'acides gras polyinsaturés oméga-3 et oméga-6 constitue au moins 20% desdits lipides.

4. Procédé selon l'une quelconque des revendications 1 à 3, où les lipides comprennent des acides gras oméga-3 et/ou oméga-6, éventuellement de l'acide docosahexaénoïque (DHA), de l'acide docosapentaénoïque (DPA), de l'acide éicosapentaénoïque (EPA) ou de l'acide arachidonique (ARA).

5. Procédé selon la revendication 2, où au moins 25% desdits lipides sont constitués d'acide docosahexaénoïque.

6. Procédé selon l'une quelconque des revendications précédentes, où ledit procédé produit en moyenne au moins 0,2 g/l/h d'acide docosahexaénoïque.

7. Procédé selon l'une quelconque des revendications précédentes, où ladite source de carbone n'est pas un alcool, éventuellement où ladite source de carbone est un glucide.

8. Procédé selon l'une quelconque des revendications précédentes, où ladite source nutritive limitante est choisie dans le groupe constitué par (i) les sources d'azote, (ii) les sources de carbone, (iii) les sources de phosphate, (iv) les sources de vitamines, telles que les sources de vitamine B12, les sources de pantothénate, les sources de thiamine, (v) le sources de métaux en traces, telles que les sources de zinc, les sources de cuivre, les sources de cobalt, les sources de nickel, les sources de fer, les sources de manganèse, les sources de molybdène, et (vi) les sources de métaux majeurs, telles que les sources de magnésium, les sources de calcium, les sources de sodium, les sources de potassium, (vii) les sources de silice, et des mélanges de celles-ci.

9. Procédé selon la revendication 8, où lesdites sources de métaux en traces et sources de métaux majeurs sont choisies dans le groupe constitué par les sels de sulfate et de chlorure de ces métaux (tels que MgSO₄·7H₂O; MnCl₂·4H₂O; ZnSO₄·7H₂O; CoCl₂·6H₂O; Na₂MoO₄·2H₂O; CuSO₄·5H₂O; NiSO₄·6H₂O; FeSO₄·7H₂O; CaCl₂; K₂SO₄; KCl; et Na₂SO₄), et des mélanges de ceux-ci.

10. Procédé selon l'une quelconque des revendications précédentes, où ladite source nutritive limitante comprend une source d'azote, par exemple un sel d'ammonium inorganique.

11. Procédé selon la revendication 10, où ladite source d'azote comprend de l'hydroxyde d'ammonium.

12. Procédé selon l'une quelconque des revendications précédentes, où lesdits microorganismes appartiennent au genre *Thraustochytrium* ou *Schizochytrium.*

13. Procédé selon l'une quelconque des revendications précédentes, où au moins 20% des lipides produits par les microorganismes sont des acides gras polyinsaturés oméga-3 et/ou oméga-6, par exemple où au moins 30% des lipides produits par les microorganismes sont des acides gras polyinsaturés oméga-3 et/ou oméga-6, par exemple où au moins 40% des lipides produits par les microorganismes sont des acides gras polyinsaturés oméga-3 et/ou oméga-6, par exemple où au moins 50% des lipides produits par les microorganismes sont des acides gras polyinsaturés oméga-3 et/ou oméga-6.

14. Procédé selon l'une quelconque des revendications précédentes, comprenant en outre la récupération des lipides à partir des microorganismes.

15. Procédé selon l'une quelconque des revendications précédentes, comprenant en outre l'utilisation des microorganismes, des lipides en étant extraits, de la biomasse restante après l'extraction des lipides ou des combinaisons de ceux-ci, comme aliment pour animaux ou complément alimentaire pour un animal sont la viande ou les produits sont consommés par l'homme, par exemple un animal dont de la viande de volaille, des fruits de mer, du boeuf, du porc ou de l'agneau, sont dérivés.
